# EUROPEAN PATENT APPLICATION

(11) **EP 4 835 465 A1**
(43) Date of publication of application: **04.11.2026**
(21) Application number: 25900001.6
(22) Date of filing: 28.09.2025
(51) Int. Cl.: G01N 30/02, G01N 30/72

(54) **METABOLIC BIOMARKER COMBINATION FOR DIAGNOSIS OF DEPRESSION**

(30) Priority: 05.12.2024 CN 202411781533; 05.12.2024 CN 202510294132
(71) Applicant: Nanjing Likang Pharmaceutical Technology Co., Ltd., Nanjing, Jiangsu 211103 (CN)
(72) Inventor: FENG, Dong, Nanjing, Jiangsu 211103 (CN); AN, Bo, Nanjing, Jiangsu 211103 (CN); BEN, Chang, Nanjing, Jiangsu 211103 (CN)
(74) Representative: dompatent
(86) International application number: PCT/CN2025/124901
(87) International publication number: WO 2026/118648

(57) **Abstract**

A metabolic biomarker combination for diagnosis of depression and a kit for diagnosis of depression, relating to the field of metabolomics. The metabolic biomarker combination consists of proline, betaine, alanine, tryptophan, kynurenine, 5-hydroxytryptamine, creatine, succinic acid, taurine, and 2-hydroxybutyric acid. The metabolic biomarker combination has high specificity, sensitivity and accuracy for diagnosis of depression, and improves the accuracy of depression diagnosis and treatment.

## Description

### TECHNICAL FIELD

The present invention relates to the field of metabolomics, and specifically, it relates to metabolic biomarker combination for diagnosis of depression.

### BACKGROUND

Depression severely affects patients' lives and work, placing a heavy burden on families and society. Approximately 15% of depressed patients die by suicide. Depression has become the second largest disease burden in China. The low recognition rate of depression in the healthcare system is mainly due to the fact that clinical diagnosis of depression primarily relies on medical history, clinical symptoms, and disease course. Currently, the diagnosis of depression mainly depends on patients' self-reported symptoms and clinical interview results, making the diagnostic process relatively subjective. This also leads to low treatment rates and high recurrence rates. Internationally accepted diagnostic criteria comprise ICD-10 and DSM-V, while domestically ICD-10 is mainly used. Such diagnostic methods rely too much on the physician's subjective judgment, require high clinical experience, and are not suitable for large-scale screening. Moreover, there is a widespread phenomenon of low clinical consultation rates among depressed patients. Therefore, for the prevention and treatment of depression, an objective testing tool is needed as a screening tool for depression management. This not only advances the "front line" of depression, proactively identifying a large number of potential depressed patients, but also effectively complements the overly subjective judgment means currently used in clinical diagnosis of depression.

Studies have shown that medication compliance in depressed patients is poor, with a one-year recurrence rate of about 33%, seriously affecting the treatment efficacy and outcome. After returning to society, patients often experience irregular relapses, making it difficult to determine the endpoint of depression treatment. Patients at different stages of depression require different treatment methods and medications. Most patients are unable to adhere to the full-course treatment strategy, which increases difficulties in clinical work. Therefore, there is a need for disease course monitoring in depressed patients, including treatment outcome and relapse prediction, which is particularly important for providing timely and effective treatment to depressed patients.

The development of multi-omics technologies provides a scientific basis for the diagnosis and disease management of depression. Metabolomics is a discipline that has emerged after genomics and proteomics and is an important part of systems biology. It has rapidly developed and penetrated many fields, such as disease diagnosis, drug research and development, toxicology, environmental science, botany, and other areas closely related to human health care. In practical clinical applications, for example, glycated hemoglobin is used to assess long-term blood glucose control and diagnose/monitor diabetes; urine glucose is used to help screen for diabetes; transaminases (ALT and AST) are used to assess liver function and detect liver injury; bilirubin is used to diagnose biliary obstruction, hepatitis, and other liver problems; creatinine is used to assess kidney function, and creatinine clearance is an important indicator of kidney health; blood urea nitrogen (BUN) is used to monitor kidney function and determine the severity of kidney disease.

In recent years, metabolomics-based studies on depression have increased. Joyce JB et al., through a metabolomics study of 298 depressed patients, showed that plasma hydroxylated sphingosine is an important predictor of treatment outcome in depression (Joyce J B. Using Multi-Domain Data to Predict Remission of Unipolar Depressive Episodes Following Antidepressant Therapy[D]. College of Medicine-Mayo Clinic, 2020.). Song et al. showed that levels of lactate, glucose, glutamine, creatinine, acetate, L-valine, L-alanine, and fatty acids were significantly decreased in serum, while acetone and choline levels were significantly increased (Song Z, Wang H, Yin X, et al. Application of NMR metabolomics to search for human disease biomarkers in blood[J]. Clinical Chemistry and Laboratory Medicine (CCLM), 2019, 57(4): 417-441.). Many studies have shown that metabolites play an important role in the mechanism, medication, and prognosis of depression. The development of kits for the screened molecular markers is of great significance for clinical application.

Machine learning models are of great importance in depression research. By analyzing large amounts of medical data, machine learning models can help physicians better diagnose depression, improving diagnostic accuracy and efficiency. In addition, machine learning can help physicians predict the possible future progression of a patient's disease and develop more effective treatment plans. By utilizing machine learning technology, researchers can also gain deeper insights into the etiology and mechanisms of depression, providing more in-depth understanding for disease prevention and treatment. Overall, machine learning can bring more breakthroughs and progress to the medical field in depression research.

Although many research institutions have studied diagnostic markers for depression, these existing studies have not provided an accurate, simple, reliable, and widely applicable clinical diagnostic method.

Patent application No. CN201610105047.3 and patent application No. CN201080046087.6 disclose the use of serum metabolic markers for diagnosing depression. However, serum sample collection is difficult and requires professional medical staff; patients cannot easily collect samples at home. Patent application No. CN201480019392.4 discloses the use of serum or urine biomarkers for diagnosis, where the set of biomarkers comprises at least cGMP, cortisol, calprotectin, thromboxane, aldosterone, HVEM, and substance P. Because the markers comprise both small molecule metabolites and large molecule proteins, multiple technology platforms (e.g., mass spectrometry and immunological methods) are required for detection, increasing detection difficulty and cost. Moreover, this invention uses serum and urine samples, which have high storage and transportation costs. Patent application No. CN202010658243.X uses filter paper strips onto which urine samples are applied for sample collection, which effectively solves some problems associated with serum and plasma, but its detection purpose is single-only predicting depression diagnosis. During the course of depression, graded diagnosis, differential identification, treatment outcome, and recurrence also have a significant impact on patients. Traditional analytical methods such as immunoassays have been the most widely used technology in clinical diagnosis. Among immunological methods, radioimmunoassay has been largely eliminated due to radioactive contamination. ELISA is manually operated, and results are susceptible to human factors. Due to the need for detection convenience and accuracy, new detection markers, methods, and tools need to be developed.

The kit provided in patent application No. CN201610105047.3 can measure the levels of 14 screened metabolic markers in blood: methionine, nicotinic acid, phosphoserine, lactic acid, glutamic acid, hypoxanthine, choline, tryptophan, 5-hydroxytryptamine, propionylcarnitine, glutarylcarnitine, estradiol, corticosterone, and estriol. Using this kit for early diagnosis of depression, the specificity reached 86.8%, sensitivity reached 96.8%, and accuracy reached 93.0%. Patent application No. CN201080046087.6 only relates to molecular markers for diagnosing depression and does not indicate the diagnostic efficacy of the metabolite combination. The literature (Doctoral Dissertation of Chongqing Medical University, author: Zheng Peng, "Screening of diagnostic markers for depression in plasma and urine based on metabolomics strategy", 2013; and Zheng P, Wang Y, Chen L, et al. Identification and validation of urinary metabolite biomarkers for major depressive disorder. Mol Cell Proteomics. 2013;12(1):207-214) discloses the use of nuclear magnetic resonance to measure urine samples from 126 depressed patients and 134 normal controls, identifying a total of 23 differential metabolites. Logistic regression analysis found that a biomarker panel consisting of five metabolites-malonate, formate, N-methylnicotinamide, M-hydroxyphenylacetate, and alanine-has potential clinical diagnostic value. This biomarker panel distinguished 82 depressed patients from 82 normal controls in the training set, with an AUC of 0.812 (95% CI: 0.829-0.961); and distinguished 44 depressed patients from 52 normal controls in the test set, with an AUC of 0.895 (95% CI: 0.829-0.961).

Based on the above research status, there is still a need to develop an improved metabolic biomarker combination for depression diagnosis, which provides superior characteristics such as higher specificity, sensitivity, accuracy, and ease of detection.

### SUMMARY OF THE INVENTION

One object of the present invention is to provide a metabolic biomarker combination for diagnosing depression. Based on this metabolic biomarker combination, an optimal diagnostic model can be selected through machine learning algorithms, and a depression diagnostic kit can be constructed.

A further object of the present invention is to provide the use of the metabolic biomarker combination in the preparation of a kit for diagnosing depression in a subject.

A further object of the present invention is to provide a detection method for testing the types and contents of metabolic biomarkers in a dried blood spot sample from a subject.

Based on the above objects, the present invention provides a metabolic biomarker combination for diagnosing depression, comprising: proline, betaine, alanine, tryptophan, kynurenine, 5-hydroxytryptamine(serotonin), creatine, succinic acid, taurine, and 2-hydroxybutyric acid. Preferably, the present invention provides a metabolic biomarker combination for diagnosing depression, wherein the metabolic biomarker combination consists of proline, betaine, alanine, tryptophan, kynurenine, 5-hydroxytryptamine, creatine, succinic acid, taurine, and 2-hydroxybutyric acid.

The present invention further provides a metabolic biomarker combination for diagnosing depression, comprising: methionine, phenylalanine, guanine, hypoxanthine, γ-aminobutyric acid, allantoin, uric acid, histidine, aspartic acid, threonine, androstenedione, cytosine, xanthine, bilirubin, pyruvic acid, linoleic acid, glutamine, serine, adipic acid, pseudouridine, tyrosine, proline, valine, ornithine, betaine, alanine, tryptophan, leucine, kynurenine, 5-hydroxytryptamine, creatine, succinic acid, taurine, 2-hydroxybutyric acid, glutamic acid, glucose, lysine, and arginine.

The present invention further provides a kit for diagnosing depression, comprising the metabolic biomarker combination. Further, the present invention provides a kit for detecting the above-mentioned metabolic markers, comprising a standard substance of the metabolic markers, a quality control substance, an isotope-labeled internal standard, a diluent, formic acid, and an ammonium acetate solution. The extraction solvent for the metabolic markers is selected from one or a mixture of water, methanol, ethanol, acetonitrile, isopropanol, and must be of chromatographic purity grade. The water, methanol, acetonitrile, isopropanol, ethanol, formic acid, and ammonium acetate used in the mobile phase must all be of chromatographic grade. The diluent is composed of one or more of methanol, water, and DMSO, and must be of chromatographic grade.

Further, the kit can be prepared according to the following method:
(1) preparing lyophilized powders of standard pure products of the metabolic markers;
(2) preparing lyophilized powders of isotope-labeled internal standards;
(3) preparing an extraction solvent for the metabolic markers;
(4) preparing a dissolution solvent for the standards and isotope-labeled internal standards;
(5) assembling the above reagents into the kit.

Further, the present invention provides a method for using the above kit, comprising:
(1) collecting a blood sample from a subject, applying the blood sample to a dried blood spot card, and drying to obtain a dried dried blood spot sample as a test sample;
(2) extracting the dried blood spot sample with a metabolite extraction solvent and an isotope-labeled internal standard solution to obtain an extract;
(3) determining the concentrations of metabolites in the dried blood spot extract using a targeted metabolomics analysis method by liquid chromatography-tandem mass spectrometry (LC-MS/MS);
(4) comparing determined metabolite concentrations with the respective population-based reference values (normal values) for each metabolite, and if the concentrations of proline, betaine, alanine, tryptophan, kynurenine, 5-hydroxytryptamine, creatine, succinic acid, taurine, and 2-hydroxybutyric acid are significantly decreased (less than 0.8 times their respective population-based reference values), then depression is determined.

In addition, the test sample of the kit provided by the present invention can also be serum and/or plasma.

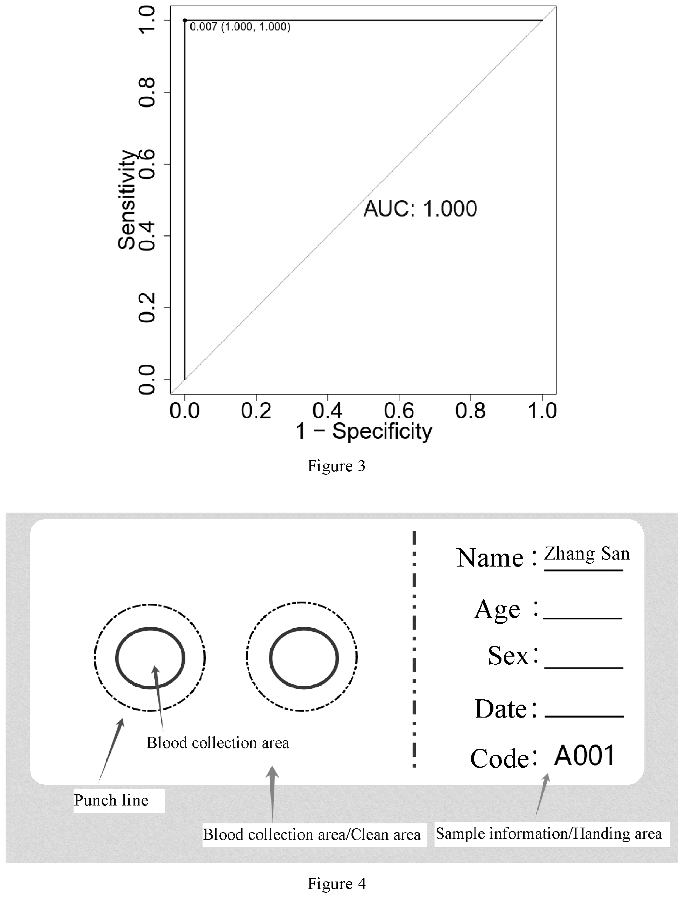

The present invention can use the following method to prepare a dried blood spot sample, comprising: (1) Collecting a dried blood spot card: the card should be clean, free from contamination and mold, and should be sealed before use. Each card should have independent identification information (name, age, sex, date, code). The card is a special card with a dedicated blood collection area. (2) The dried blood spot card is divided into two parts: one part is a blood collection area, having an inner circle with a radius of 3 mm and an outer circle with a radius of 5 mm; this area must not be touched; the other part is the handling area, which must be handled with clean medical gloves. (3) The dried blood spot card comprises an antioxidant (VC) and an enzyme inactivator, 1-aminobenzotriazole (ABT). (4) Capillary blood and venous blood can both be used for blood sampling. When collecting from capillaries, such as from a fingertip, the collection site should be cleaned in advance, and non-volatile disinfectants such as iodophor should not be used. One drop of blood is applied to each inner circle of the dried blood spot card, ensuring that the entire circle is covered and the filter paper is completely saturated, while avoiding the blood from diffusing into the outer circle. The collection process should be carried out under protected light conditions; if such conditions are not available, exposure to strong light should be avoided to prevent any adverse effects on light-sensitive substances. (5) After blood spot collection, prolonged exposure to air should be avoided. Drying can be accelerated by nitrogen blowing or by using vacuum equipment. The collected dried blood spots are rapidly immersed in liquid nitrogen to inactivate enzymes, and then air-dried. The cards should not be stacked on top of each other; they should be individually packaged in separate sealed bags and stored in a -80°C freezer. Figure 4 provides a schematic example of a dried blood spot card.

In the present invention, the dried blood spot sample is processed as follows: a 10 mm diameter disc is punched from the sample area along the outer circle line of the card using a punch, and the disc is placed into a 1.5 mL centrifuge tube. Then, 500 mL of eluent/precipitant comprising an internal standard is added. The tube is vortexed for approximately 5 minutes. The sample is centrifuged at 15,000 g for 10 minutes, and the supernatant is collected. The supernatant is then subjected to a second centrifugation (or filtered through a membrane filter), and the resulting supernatant is collected for injection. During the punching step, holes are punched strictly along the outer circle of the card to prevent cross-contamination between samples, which could otherwise compromise the detection results.

The metabolic markers of the present invention can be used for the diagnosis of depression or affective disorders in a subject.

In the present invention, the depression is selected from the group consisting of mild depression, moderate-to-severe depression, and bipolar depression. For brevity, other parts of the present invention refer to "depression", and those skilled in the art will understand that the principles and purposes of the described technical solutions also apply to other types of affective disorders.

In the present invention, "metabolic marker" refers to a unique biological or biologically derived indicator of a process, event, or condition. Biomarkers can be used for depression diagnosis. The biomarkers provided by the present invention have high specificity, sensitivity, and accuracy for diagnosing depression. The present invention enables depression diagnosis to no longer rely entirely on the subjective judgment of clinical experience and questionnaire scales, greatly improving diagnostic and treatment accuracy.

In the present invention, "subject" is a human.

The inventors of the present invention, based on targeted metabolomics analysis using liquid chromatography-tandem mass spectrometry (LC-MS/MS), have identified, by detecting samples from healthy subjects and patients with depression, a plurality of biomarkers useful for diagnosing depression, which can achieve high specificity, sensitivity, and accuracy.

The present invention detects metabolites in dried blood spot cards, which not only effectively avoids reliance on subjective physician judgment but also assists in depression diagnosis, which is of great clinical significance. The sample is stored on a dried blood spot card, which has advantages such as easy preparation and collection, convenient transportation, and room temperature storage. The present invention uses liquid chromatography-tandem mass spectrometry (LC-MS/MS) for targeted quantification of the biomarkers to be tested, providing accurate and convenient sample detection.

In diagnosing depression, the present invention uses a generalized linear model (GLM), with an area under the curve (AUC) value of 1, an accuracy of 99.30%, a sensitivity of 100%, and a specificity of 98.96%. This indicates that the metabolic biomarker combination and the constructed model provided by the present invention have excellent diagnostic value, significantly superior to previous similar reports. The metabolic biomarker combination provided by the present invention is innovative, particularly because no previous studies have shown that 2-hydroxybutyric acid is associated with depression diagnosis. 2-Hydroxybutyric acid is related to insufficient energy metabolism and impaired glucose regulation. Some studies have shown that 2-hydroxybutyric acid is significantly elevated in patients with bipolar disorder (Simić K, Miladinović Z, Todorović N, et al. Metabolomic Profiling of Bipolar Disorder by 1H-NMR in Serbian Patients[J]. Metabolites, 2023, 13(5): 607.). However, no study has shown that 2-hydroxybutyric acid can serve as a molecular marker for diagnosing depression. Adding 2-hydroxybutyric acid as a diagnostic molecular marker in the present application is innovative.

### BRIEF DESCRIPTION OF THE DRAWINGS

Figure 1 shows chromatograms of metabolic markers.
Figure 2 shows PCA and OPLS-DA analyses of two groups of samples: healthy individuals and depressed patients.
Figure 3 shows the ROC curve of the generalized linear model (GLM) distinguishing healthy individuals from depressed patients.
Figure 4 is a schematic diagram illustrating an example of a dried blood spot card.

### DETAILED DESCRIPTION OF THE INVENTION

To make the objectives, technical solutions, and effects of the present invention clearer and more comprehensible, the present invention is further described in detail below with reference to the accompanying drawings and examples. It should be understood that the specific embodiments described herein are only for explaining the present invention and are not intended to limit the present invention.

The present invention provides a set of metabolic markers that can be used for diagnosing depression. The metabolic markers comprise: proline, betaine, alanine, tryptophan, kynurenine, 5-hydroxytryptamine, creatine, succinic acid, taurine, and 2-hydroxybutyric acid. Alternatively, the metabolic markers comprise: methionine, phenylalanine, guanine, hypoxanthine, γ-aminobutyric acid, allantoin, uric acid, histidine, aspartic acid, threonine, androstenedione, cytosine, xanthine, bilirubin, pyruvic acid, linoleic acid, glutamine, serine, adipic acid, pseudouridine, tyrosine, proline, valine, ornithine, betaine, alanine, tryptophan, leucine, kynurenine, 5-hydroxytryptamine, creatine, succinic acid, taurine, 2-hydroxybutyric acid, glutamic acid, glucose, lysine, and arginine. The present invention preferably uses targeted mass spectrometry technology, but other measurement techniques such as immunoassay and chemiluminescence can also be used for the determination of the metabolic markers of the present invention.

The metabolic markers of the present invention are screened by the following method:
(1) selecting depressed patients and healthy controls, respectively, and taking plasma samples to prepare dried blood spots, from which metabolites are extracted;
(2) determining the contents of major metabolites in the dried blood spots using metabolomics methods, specifically liquid chromatography-tandem mass spectrometry (LC-MS/MS);
(3) performing orthogonal partial least squares discriminant analysis (OPLS-DA) and variable importance in projection (VIP) analysis on the obtained data to identify metabolites with significant differences between the two groups as candidate metabolic markers;
(4) further screening to determine a metabolic marker combination with high accuracy and specificity using receiver operating characteristic (ROC) curves.

These metabolic markers can be detected in the blood sample of a subject. The present invention preferably uses a plasma sample from a subject and prepares dried blood spots as the test sample.

During measurement, metabolites are first extracted from the dried blood spots. The present invention uses, but is not limited to, water, methanol, ethanol, acetonitrile, isopropanol, or a mixture of the above solvents to extract metabolites from dried blood spots, optionally comprising the corresponding isotope-labeled internal standards for each biomarker. Preferably, a 5-15% (v/v) methanol aqueous solution is used to extract metabolites from dried blood spots. Preferably, extraction is performed at room temperature (20-35°C) with shaking for 20-40 minutes, and the dried blood spot extract is used for subsequent measurement.

In the measurement, the present invention preferably uses a derivatization method to detect the metabolic markers. Derivatization methods include, but are not limited to, dansyl chloride, phenyl isothiocyanate, 6-aminoquinolyl-N-hydroxysuccinimidyl carbamate, etc. Preferably, dansyl chloride is used for derivatization. Preferably, the supernatant of the dried blood spot extract is taken, and sodium bicarbonate solution and a derivatization reagent are added. The mixture is vortexed for a period of time to allow sufficient reaction. Preferably, the dansyl chloride-derivatized metabolic markers are extracted with ethyl acetate. The upper organic phase of the extract is transferred to a suitable container and evaporated to dryness under nitrogen. After reconstitution with a methanol aqueous solution, detection is performed.

The present invention preferably uses liquid chromatography-tandem mass spectrometry (LC-MS/MS) for the detection and analysis of the above diagnostic marker combination.

Preferably, the liquid chromatography conditions are: a chromatographic column (can be a C8 or C18 silica gel column), mobile phase (can be acetonitrile aqueous solution, formic acid aqueous solution, methanol aqueous solution, acetonitrile-isopropanol, methanol-isopropanol solution, acetic acid aqueous solution, ammonium acetate aqueous solution), and the elution program is gradient elution with mobile phase A and mobile phase B in a certain ratio.

In a specific example of the present invention, the liquid chromatography conditions are: a ZORBAX RR Extend-C18, 80Å, 4.6×150 mm, 3.5 µm column. Liquid chromatography conditions: injection volume: 5 µL; flow rate: 0.6 mL/min; column temperature: 40°C; autosampler temperature: 4°C; mobile phase A: 0.1% (v/v) formic acid in water; mobile phase B: methanol; elution program: 0-1 min, linear change from 10% B to 40% B; 1-1.5 min, linear change from 40% B to 50% B; 1.5-2.0 min, linear change from 50% B to 60% B; 2.0-3.2 min, equilibration at 80% B; 3.2-3.6 min, change from 80% B to 10% B; 3.7-4.5 min, equilibration at 10% B.

Preferably, a triple quadrupole mass spectrometer is used for detection. Electrospray ionization (ESI) source is used, with positive and negative ion switching mode, scan time of 0 min to 4.5 min; CUR: 20; CAD: Medium; IS: 5000; TEM: 500; GS1: 30; GS2: 50; Interface heater (ihe): on; DP: ±36; CE: ±26; EP: ±15; CXP: ±10. Dwell Time: 20 ms; Resolution Q1: Unit; Resolution Q3: Unit; Pause between mass: 5.007 ms.

After data acquisition, each diagnostic metabolic marker is quantified using specific ion pairs. The peak area of the acquired signal is compared with the standard curve of the corresponding standard, and the concentration of the metabolic marker is obtained after correction using the added internal standard solution. The content of each metabolic marker in the original blood is thus determined.

A specific embodiment of the present invention is a diagnostic kit for depression, comprising: 1 mg of lyophilized powder of standard substances of the metabolic markers; 1 mg of quality control standards of the markers; a lyophilized powder mixture comprising at least three stable isotope-labeled internal standards, wherein isotope labels are selected from the group consisting of ¹³C, ¹⁵N, and ²H (deuterium); 5 mL of methanol-water-DMSO solution (diluent) for dissolving standard powder and isotope-labeled internal standard powder; 1 mL of formic acid solution; 5 mol/L ammonium acetate solution for adjusting the mobile phase pH; a methanol solution as a metabolite extraction solvent; and one analytical chromatographic column.

Preferably, in the diagnostic kit for depression, the metabolic markers comprise: proline, betaine, alanine, tryptophan, kynurenine, 5-hydroxytryptamine, creatine, succinic acid, taurine, and 2-hydroxybutyric acid. Alternatively, the metabolic markers comprise: methionine, phenylalanine, guanine, hypoxanthine, γ-aminobutyric acid, allantoin, uric acid, histidine, aspartic acid, threonine, androstenedione, cytosine, xanthine, bilirubin, pyruvic acid, linoleic acid, glutamine, serine, adipic acid, pseudouridine, tyrosine, proline, valine, ornithine, betaine, alanine, tryptophan, leucine, kynurenine, 5-hydroxytryptamine, creatine, succinic acid, taurine, 2-hydroxybutyric acid, glutamic acid, glucose, lysine, and arginine.

The preparation method of the above-mentioned diagnostic kit for depression comprises the following specific preparation steps:
(1) Sample preparation: a sample to be analyzed is collected, ensuring that the sample is within the required concentration range. The sample is diluted as necessary (typically with a mobile phase). The sample is filtered to remove possible particulate matter, typically using a 0.45 µm or 0.2 µm filter membrane.
(2) Mobile phase selection: the metabolite extract is injected via an autosampler into an Extend C18 chromatographic column (ZORBAX RR Extend-C18, 80Å, 4.6 × 150 mm, 3.5 µm, USA) to separate metabolites in the blood sample. The specific liquid chromatography conditions are as follows: injection volume: 5 µL; flow rate: 0.6 mL/min; column temperature: 40 °C; autosampler temperature: 4 °C; mobile phase A: 0.1% (v/v) formic acid in water; mobile phase B: methanol. The elution program is as follows: 0-1 min, linear gradient from 10% B to 40% B; 1-1.5 min, linear gradient from 40% B to 50% B; 1.5-2.0 min, linear gradient from 50% B to 60% B; 2.0-3.2 min, equilibration at 80% B; 3.2-3.6 min, from 80% B to 10% B; 3.7-4.5 min, equilibration at 10% B.
(3) Instrument setup: each component of the HPLC system, comprising the pump, autosampler, chromatographic column, and detector, is inspected to ensure proper operation. The chromatographically separated metabolites are introduced into a triple quadrupole mass spectrometer, and the metabolites are scanned and detected in multiple reaction monitoring (MRM) mode using an electrospray ionization (ESI) source in positive/negative ion polarity switching mode, with a scan time of 0-4.5 min. CUR: 20; CAD: Medium; IS: 5000; TEM: 500; GS1: 30; GS2: 50; Interface heater (ihe): on; DP: ±36; CE: ±26; EP: ±15; CXP: ±10. Dwell Time: 20 ms; Resolution Q1: Unit; Resolution Q3: Unit; Pause between mass: 5.007 ms.
(4) Sample injection: the prepared sample is introduced into the system using an autosampler. A typical injection volume is 10-100 µL. Smooth liquid flow of the sample is ensured, and the injection time is recorded.
(5) After data acquisition, each diagnostic metabolite marker is quantified using a specific ion pair. The peak area of the acquired signal is compared with a working curve of a corresponding standard, and the internal standard solution is added for correction to obtain the concentration values of the metabolic markers.

The quantitative values of the metabolite markers obtained from the above test are compared with the population-based reference values of each metabolite to perform a corresponding depression diagnosis and risk assessment. The measured metabolite concentrations are compared with the population-based reference values of each metabolite. If the concentrations of proline, betaine, alanine, tryptophan, kynurenine, 5-hydroxytryptamine, creatine, succinic acid, taurine, and 2-hydroxybutyric acid are significantly decreased (less than 0.8 times their respective population-based reference values), then depression is determined.

### EXAMPLES

The present invention is further described below with reference to specific examples.

### 1. Inclusion of samples

In this example, the following inclusion criteria were adopted for the depression sample group: (1) all enrolled depressed patients met the ICD-10 diagnostic criteria; (2) 17-item Hamilton Depression Rating Scale (HDRS) score was ≥18; (3) patients with first-episode depression who had not taken any antidepressant medication; (4) age between 18 and 60 years (>18 years and <60 years).

Meanwhile, the following exclusion criteria were adopted for the depression sample group: (1) patients with other current psychiatric disorders or a history of other psychiatric disorders; (2) patients with combined organic brain diseases and severe brain trauma, or combined heart, liver, kidney diseases, diabetes, and other severe physical diseases; (3) patients with abnormal routine laboratory tests (blood routine, liver function, urinalysis); (4) female subjects who were pregnant, lactating, or menstruating; (5) subjects with a history of drug or substance abuse.

The inclusion criteria for the healthy control group were as follows: no history of neuropsychiatric diseases, no history of drug abuse or dependence, and no systemic somatic diseases; no significant abnormalities were observed in routine laboratory tests.

### 2. Preparation of dried blood spots

Dried blood spot cards were prepared: the cards were kept clean, free from contamination and mold, and were sealed before use. Each card was provided with unique identification information (name, age, sex, code). The cards were provided with blood collection areas. Each dried blood spot card was divided into two parts: the first part was the blood collection area, which had an inner circle with a radius of 3 mm and an outer circle with a radius of 5 mm; this area was prohibited from being touched; the second part was the handling area, which was required to be held only with clean medical gloves. The dried blood spot cards comprised an antioxidant (vitamin C) and an enzyme inactivator, 1-aminobenzotriazole (ABT).

Blood sampling was carried out using capillary blood and venous blood. A blood sample was collected from a fingertip capillary. The fingertip was cleaned in advance, and non-volatile disinfectants such as iodophor were not used. Then, one drop of blood was applied to each inner circle of the dried blood spot card, ensuring that the entire circle was covered and that the filter paper was fully penetrated, while avoiding blood soaking into the outer circle. The collection process was performed away from strong light to avoid affecting photosensitive substances. After the blood spots were collected, prolonged exposure to air was avoided. The spots were dried rapidly by means of nitrogen blowing or a vacuum instrument. The collected dried blood spots were quickly immersed in liquid nitrogen to inactivate enzymes, and then were air-dried. The cards were not stacked; they were individually packaged in separate sealed bags and then stored in a freezer at -80°C.

### 3. Extraction of biomarkers from the dried blood spot cards

(1) A punch was used to cut a small disc of 10 mm in diameter along the outer circle line from the dried blood spot sample area. The disc was placed into a 1.5 mL centrifuge tube, and then 500 mL of an elution/precipitation solution containing internal standards was added. The mixture was vortexed for approximately 5 minutes. The mixture was centrifuged at 15,000g for 10 minutes. The supernatant was collected and subjected to a second centrifugation (or filtration through a membrane filter), and the final supernatant was taken for injection.
(2) 500 µL of the methanol-water solution (metabolite extraction solvent) from the diagnostic kit for depression was added. The mixture was shaken at 1450 rpm for 45 minutes at 20°C.
(3) 10 µL of the extract was taken and mixed with 90 µL of water. Then, 200 µL of internal standard working solution, 50 µL of 1 M NaHCO₃ solution, and 100 µL of 1 mg/mL derivatization reagent were added sequentially. The mixture was shaken at 1450 rpm for 30 minutes at 30°C.

### 4. Detection of biomarkers in the dried blood spot cards

A targeted metabolomics analysis method based on liquid chromatography-tandem mass spectrometry (LC-MS/MS) was used. The detected metabolites comprised: methionine, phenylalanine, guanine, hypoxanthine, γ-aminobutyric acid, allantoin, uric acid, histidine, aspartic acid, threonine, androstenedione, cytosine, xanthine, bilirubin, pyruvic acid, linoleic acid, glutamine, serine, adipic acid, pseudouridine, tyrosine, proline, valine, ornithine, betaine, alanine, tryptophan, leucine, kynurenine, 5-hydroxytryptamine, creatine, succinic acid, taurine, 2-hydroxybutyric acid, glutamic acid, glucose, lysine, and arginine.

Specific steps:
(1) Sample preparation: a sample to be analyzed was collected, ensuring that the sample was within the required concentration range. The sample was diluted as necessary (typically with a mobile phase). The sample was filtered to remove possible particulate matter, typically using a 0.45 µm or 0.2 µm filter membrane.
(2) Mobile phase selection: the metabolite extract was injected via an autosampler into an Extend C18 chromatographic column (ZORBAX RR Extend-C18, 80Å, 4.6 × 150 mm, 3.5 µm, USA) to separate metabolites in the blood sample. The specific liquid chromatography conditions were as follows: injection volume: 5 µL; flow rate: 0.6 mL/min; column temperature: 40 °C; autosampler temperature: 4 °C; mobile phase A: 0.1% (v/v) formic acid in water; mobile phase B: methanol. The elution program was as follows: 0-1 min, linear gradient from 10% B to 40% B; 1-1.5 min, linear gradient from 40% B to 50% B; 1.5-2.0 min, linear gradient from 50% B to 60% B; 2.0-3.2 min, equilibration at 80% B; 3.2-3.6 min, from 80% B to 10% B; 3.7-4.5 min, equilibration at 10% B.
(3) Instrument setup: each component of the HPLC system, comprising the pump, autosampler, chromatographic column, and detector, was inspected to ensure proper operation. The chromatographically separated metabolites were introduced into a triple quadrupole mass spectrometer, and the metabolites were scanned and detected in multiple reaction monitoring (MRM) mode using an electrospray ionization (ESI) source in positive/negative ion polarity switching mode, with a scan time of 0-4.5 min. CUR: 20; CAD: Medium; IS: 5000; TEM: 500; GS1: 30; GS2: 50; Interface heater (ihe): on; DP: ±36; CE: ±26; EP: ±15; CXP: ±10. Dwell Time: 20 ms; Resolution Q1: Unit; Resolution Q3: Unit; Pause between mass: 5.007 ms.
(4) Sample injection: the prepared sample was introduced into the system using an autosampler. A typical injection volume was 10-100 µL. Smooth liquid flow of the sample was ensured, and the injection time was recorded.
(5) After data acquisition, each diagnostic metabolite marker was quantified using a specific ion pair. The peak area of the acquired signal was compared with a working curve of a corresponding standard, and the internal standard solution was added for correction to obtain the concentration values of the metabolic markers. The measured metabolite concentrations were then compared between the depression sample group and the healthy control group.

### 4. Stability experiment

After the dried blood spot papers were air-dried, they were stored under three different conditions: at -80°C, at -20°C, and at room temperature. The contents of substances in the dried blood spots were determined by liquid chromatography-mass spectrometry. The samples stored at -80°C were measured once per month, the samples stored at -20°C were measured once every half month, and the samples stored at room temperature were measured every other day. The relative standard deviation (RSD) was calculated. Using the initial mean value as the reference, the recovery rate was calculated. The results showed that the precision was within 10%, and the recovery rates were between 80% and 110%. It was demonstrated that the dried blood spot papers could be stably stored for at least one year at -80°C, for at least three months at -20°C, and for 15 days at room temperature.

### 5. Detection results

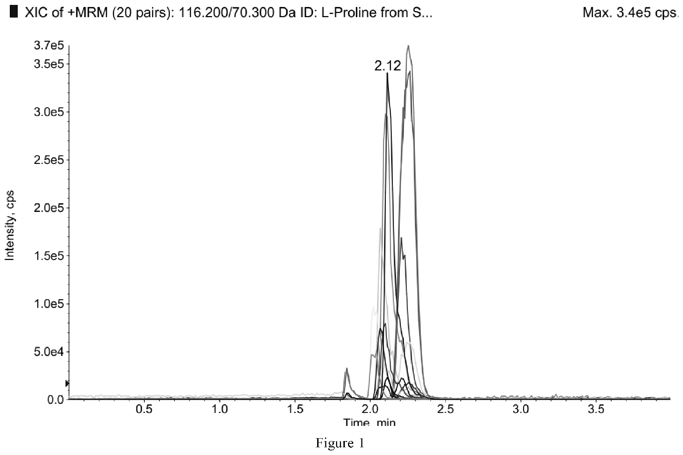

Dried blood spot samples from 150 healthy individuals were collected, and the quantification of metabolic substances in the dried blood spot samples was performed. The population-based reference ranges for the markers were established. The chromatograms of the metabolic markers are shown in Figure 1. During the analysis of the dried blood spot samples, one quality control (QC) sample was inserted after every 10 study samples to evaluate the variability of the derivatization process and the instrumental analysis. The QC samples were essential to ensure the reproducibility, reliability, accuracy, and robustness of the metabolite quantification analysis.

Meanwhile, samples from 150 healthy individuals and 60 depressed patients were collected and analyzed. The test results of the samples indicated that the differences in the levels of the following metabolites could effectively distinguish healthy individuals from depressed patients: proline, betaine, alanine, tryptophan, kynurenine, 5-hydroxytryptamine, creatine, succinic acid, taurine, and 2-hydroxybutyric acid. That is, the concentrations of these metabolic biomarkers in the depression sample group were significantly lower than those in the healthy control group (Table 1).

**Table 1. Differentially expressed metabolites between the depression group and the healthy group**

| Metabolite | Depressed patients | Healthy individuals | FC value | VIP value | P value |
|---|---|---|---|---|---|
| Proline (µmol/L) | 81.48±15.57 | 98.71±16.94 | 0.83 | 1.56 | <0.001 |
| 5-Hydroxytryptamine (ng/mL) | 43.00±6.71 | 100.94±9.10 | 0.43 | 5.61 | <0.001 |
| Creatine (mg/dL) | 0.95±0.23 | 0.81±0.21 | 1.17 | 3.15 | <0.001 |
| Betaine (ng/mL) | 5674±1840 | 5017±1679 | 1.13 | 2.42 | 0.0175 |
| Alanine (µmol/L) | 328.37±105.55 | 375.79±88.67 | 0.87 | 3.71 | 0.0024 |
| Tryptophan (µmol/L) | 56.11±6.87 | 89.48±9.41 | 0.63 | 4.11 | <0.001 |
| Kynurenine (µmol/L) | 2.75±0.82 | 2.23±0.45 | 1.23 | 1.87 | <0.001 |
| Succinic acid (ng/mL) | 334.02±100.8 | 519.31±141.87 | 0.64 | 4.30 | <0.001 |
| Taurine (ng/mL) | 14451±3199 | 11814±4429 | 1.22 | 1.13 | <0.001 |
| 2-Hydroxybutyric acid (ng/mL) | 6490±2624 | 4946±1883 | 1.31 | 1.91 | <0.001 |

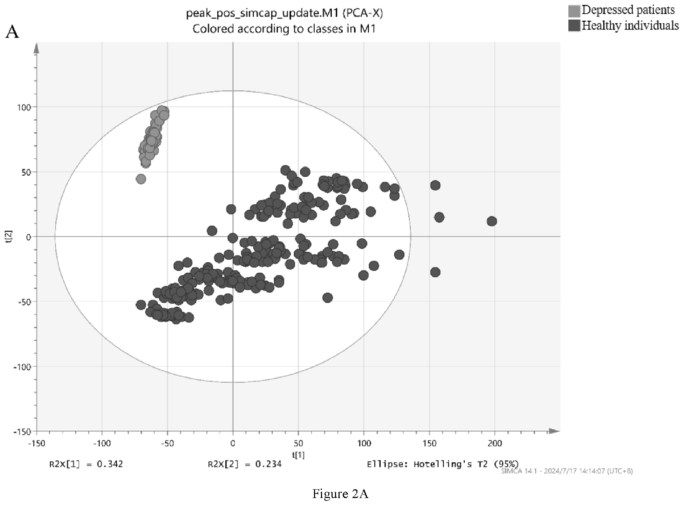

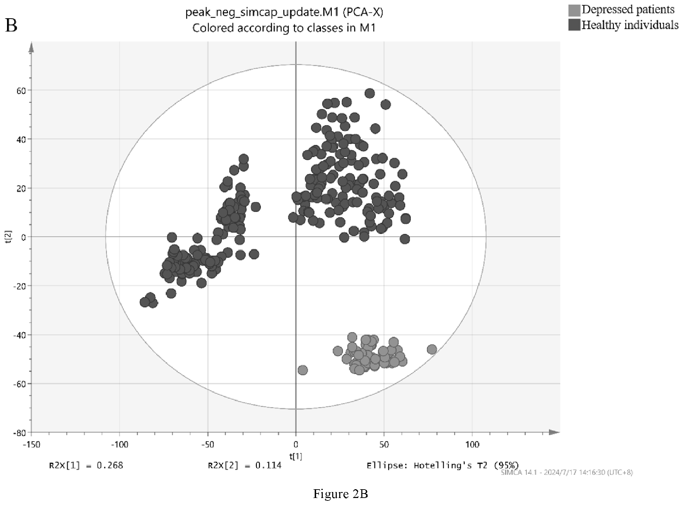

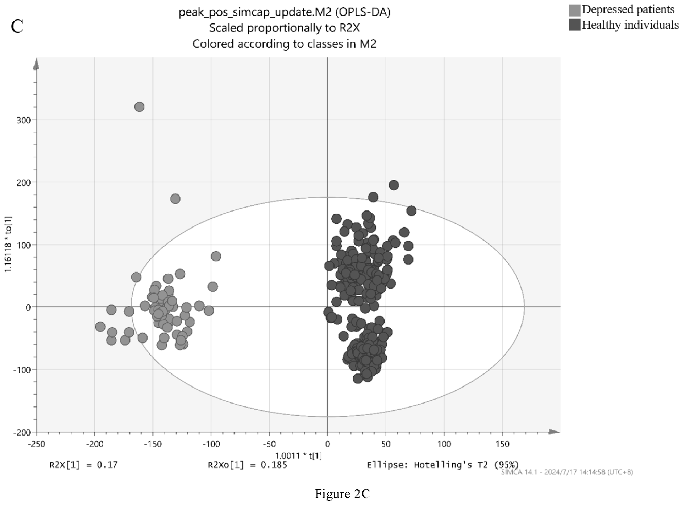

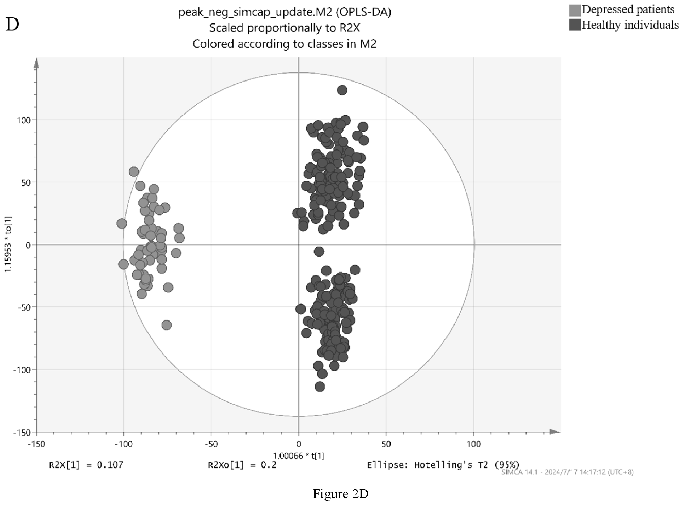

Figures 2A and 2B are principal component analysis (PCA) score plots, which show that the QC samples cluster tightly relative to the other dried blood spot samples, indicating good repeatability of the method of the present example. The QC samples here are BSA (bovine serum albumin solution). Orthogonal partial least squares discriminant analysis (OPLS-DA) was performed. As shown in Figures 2C and 2D, the normal and depression samples were well separated, indicating significant metabolic differences between depressed patients and normal individuals.

Different machine learning algorithms were further used to construct models. The machine learning algorithms used comprised generalized linear model, linear discriminant analysis, K-nearest neighbors algorithm, logistic regression, lasso regression, decision tree, artificial neural network, support vector machine, extreme gradient boosting, random forest, principal component analysis, Bayesian network, and linear regression. Among them, the operating procedures of the generalized linear model are as follows: an appropriate link function and distribution are selected, and the model is fitted using maximum likelihood estimation (MLE). This can be implemented using statistical software, e.g., the glm function in R. The operating procedures of linear discriminant analysis (LDA) are as follows: the mean and covariance matrix of each class are calculated, and eigenvalues and eigenvectors are solved to obtain the optimal separation. This is typically implemented in R or Python's sklearn. The operating procedures of the K-nearest neighbors (KNN) algorithm are as follows: a K value is chosen, distances between the point to be classified and all points in the training data are calculated, the distances are sorted, the K nearest neighbors are selected, and the class is determined by majority voting. In Python, KNeighborsClassifier from sklearn can be used. The operating procedures of logistic regression are as follows: the logistic function (sigmoid) is used for modeling, and parameters are optimized using maximum likelihood estimation. This can be implemented via glm in R or sklearn in Python. The operating procedures of lasso regression are as follows: an L1 regularization term is added to linear regression, and the model is implemented using coordinate descent or least squares. The Lasso class in Python's sklearn is available for this purpose. The operating procedures of decision trees are as follows: the best feature is recursively selected for node splitting until a stopping condition is met. This can be implemented using DecisionTreeClassifier or DecisionTreeRegressor from sklearn. The operating procedures of artificial neural networks are as follows: the network structure (number of layers, number of neurons) is designed, and parameters are optimized using the backpropagation algorithm. Deep learning frameworks such as TensorFlow or PyTorch can be used. The operating procedures of support vector machines (SVM) are as follows: an appropriate kernel function is selected, and the objective function is optimized to find the optimal hyperplane. This can be implemented using SVC from sklearn. The operating procedures of extreme gradient boosting (XGBoost) are as follows: gradient boosting trees are constructed, and the loss function is optimized iteratively. This can be implemented using the xgboost library, with hyperparameters tuned for optimization. The operating procedures of random forests are as follows: multiple decision trees are constructed, and different training sets are generated using Bootstrap sampling. The predictions of each tree are finally combined by voting or averaging. This can be implemented using RandomForestClassifier or RandomForestRegressor from sklearn. The operating procedures of principal component analysis (PCA) are as follows: the data are standardized, the covariance matrix is calculated, eigenvalues and eigenvectors are obtained, and the top K eigenvectors are selected as principal components. PCA can be implemented using PCA from sklearn. The operating procedures of Bayesian networks are as follows: the network structure is defined, probabilities are updated using Bayes' theorem, and parameter learning is typically performed using maximum likelihood estimation or Bayesian estimation. This can be implemented using the pgmpy library. The operating procedures of linear regression are as follows: a linear equation is established, and the parameters are fitted using the least squares method. This can be implemented using lm in R or LinearRegression in Python's sklearn.

The results showed that, as shown in Table 2, the best model was the generalized linear model (GLM). The receiver operating characteristic (ROC) curve of the significantly changed metabolites had an area under the curve (AUC) value of 1, an accuracy of 99.30%, a sensitivity of 100%, and a specificity of 98.96% (see Figure 3). This indicates that the model has excellent diagnostic value. The combination of metabolites proline, betaine, alanine, tryptophan, kynurenine, 5-hydroxytryptamine, creatine, succinic acid, taurine, and 2-hydroxybutyric acid exhibited excellent specificity and sensitivity.

**Table 2. Comparative analysis of results of different models for depression diagnosis**

| Model | AUC value | Accuracy (%) | Sensitivity (%) | Specificity (%) |
|---|---|---|---|---|
| Generalized Linear Model | 1.000 | 99.30% | 100.00% | 98.96% |
| Linear Discriminant Analysis | 0.9982 | 98.60% | 97.92% | 100.00% |
| K-Nearest Neighbors Algorithm | 0.9576 | 94.41% | 97.92% | 87.23% |
| Logistic Regression | 0.9976 | 97.90% | 97.92% | 97.87% |
| Lasso Regression | 0.9981 | 97.20% | 98.96% | 93.62% |
| Decision Tree | 0.9983 | 97.20% | 96.87% | 97.87% |
| Artificial Neural Network | 0.9749 | 93.71% | 93.75% | 93.62% |
| Support Vector Machine | 0.9978 | 97.20% | 97.92% | 95.74% |
| Extreme Gradient Boosting | 0.9989 | 99.30% | 98.96% | 100.00% |
| Random Forest | 0.9996 | 97.90% | 96.87% | 100.00% |
| Principal Component Analysis | 0.9980 | 97.90% | 97.92% | 97.87% |
| Bayesian Network | 0.9844 | 97.90% | 96.87% | 100.00% |
| Linear Regression | 0.9953 | 99.30% | 98.96% | 100.00% |

Dried blood spot samples were randomly obtained from outpatients or inpatients of a certain hospital, as well as from recruited healthy controls (total of 300 samples, all samples were collected with clear diagnostic data based on ICD-10 criteria by professional physicians, and informed consent was obtained). The biomarkers (proline, betaine, alanine, tryptophan, kynurenine, 5-hydroxytryptamine, creatine, succinic acid, taurine, and 2-hydroxybutyric acid) were directly detected according to the method of Example 1, using the ratio of the ion abundance of the biomarkers to the internal standard. The data were input into the diagnostic model established in Example 1 to obtain diagnostic results. These diagnostic results were compared with the results of depression diagnosis by professional physicians on the 300 samples using ICD-10. The results are shown in Table 3.

**Table 3. Evaluation of the efficacy of the metabolic marker kit for diagnosing depression**

| Diagnostic model result | ICD-10 diagnosis result | |
|---|---|---|
| | Normal individuals | Depressed patients |
| Normal individuals (120 cases) | 120 | 0 |
| Depressed patients (180 cases) | 0 | 180 |
| Total | 120 | 180 |

As shown in Table 2, using the marker combination of Example 1 and the diagnostic model established thereby, the accuracy for depression diagnosis was 100%, the sensitivity was 100%, and the specificity was 100%.

The embodiments of the present invention have been described in detail above, but the present invention is not limited to the described embodiments. For those skilled in the art, various changes, modifications, substitutions, and variations to these embodiments without departing from the principle and spirit of the present invention still fall within the protection scope of the present invention.

## Claims

1. A metabolic biomarker combination for diagnosing depression, wherein the metabolic biomarker combination consists of proline, betaine, alanine, tryptophan, kynurenine, 5-hydroxytryptamine, creatine, succinic acid, taurine, and 2-hydroxybutyric acid.

2. The metabolic biomarker combination of claim 1, wherein the sample for detecting the metabolic biomarkers is human serum, plasma, or dried blood spot.

3. A kit for diagnosing depression, wherein the kit comprises reagents for determining the metabolic biomarker combination of any one of claims 1-2.

4. The kit of claim 3, wherein the kit further comprises: a standard substance of the metabolic biomarker combination, a quality control substance, an isotope-labeled internal standard, a diluent, formic acid, and an ammonium acetate solution.

5. The kit of claim 4, wherein the kit comprises: 1 mg of lyophilized powder of standard substances of the metabolic markers; 1 mg of quality control standards of the markers; a lyophilized powder mixture comprising at least three stable isotope-labeled internal standards, wherein isotope labels are selected from the group consisting of ¹³C, ¹⁵N, and ²H (deuterium); 5 mL of methanol-water-DMSO solution (diluent) for dissolving standard powder and isotope-labeled internal standard powder; 1 mL of formic acid solution; 5 mol/L ammonium acetate solution for adjusting the mobile phase pH; a methanol solution as a metabolite extraction solvent; and one analytical chromatographic column.

6. The kit of claim 5, wherein a method of using the kit comprises:
(1) collecting a blood sample from a subject, applying the blood sample to a dried blood spot card, and drying to obtain a dried dried blood spot sample as a test sample;
(2) extracting the dried blood spot sample with a metabolite extraction solvent and an isotope-labeled internal standard solution to obtain an extract;
(3) determining the concentrations of metabolites in the dried blood spot extract using a targeted metabolomics analysis method by liquid chromatography-tandem mass spectrometry (LC-MS/MS);
(4) comparing determined metabolite concentrations with the respective population-based reference values for each metabolite, and if the concentrations of proline, betaine, alanine, tryptophan, kynurenine, 5-hydroxytryptamine, creatine, succinic acid, taurine, and 2-hydroxybutyric acid are less than 0.8 times their respective population-based reference values, then depression is determined.

7. Use of the metabolic biomarker combination of claim 1 in the preparation of a kit for diagnosing depression.

8. The use of claim 7, wherein the depression is selected from the group consisting of mild depression, moderate-to-severe depression, and bipolar depression.
